# EUROPEAN PATENT APPLICATION

(11) **EP 3 954 396 A1**
(43) Date of publication of application: **16.02.2022**
(21) Application number: 20194456.8
(22) Date of filing: 03.09.2020
(51) Int. Cl.: A61L 2/18, A61L 2/22, A61L 9/14

(54) **METHOD, APPARATUS AND SYSTEM FOR DISINFECTING AIR AND/OR SURFACES**

(30) Priority: 10.08.2020 EP 20190355
(71) Applicant: Proteck Germany GmbH, 14641 Nauen (DE)
(72) Inventor: Bone-Winkel, Thomas, 14641 Nauen (DE); Singh, Gundeep, 14641 Nauen (DE)
(74) Representative: RGTH

(57) **Abstract**

To provide a method for disinfecting air and/or surfaces which can be carried out truly continuously, in particular for essentially 24 hours a day, with people and/or animals present, a method for disinfecting air and/or surfaces is proposed, wherein a disinfectant is dispersed continuously into a volume and/or area to be disinfected, wherein the disinfectant comprises electrolyzed and/or sterilized water containing hypochlorous acid, and wherein the disinfectant is dispersed at a dispersion rate between 0.5 ml/m³/h and 12 ml/m³/h.

## Description

The present invention relates to a method for disinfecting air and/or surfaces, wherein a disinfectant is dispersed continuously into a volume and/or area to be disinfected, wherein the disinfectant comprises electrolyzed and/or sterilized water containing hypochlorous acid. The present invention further relates to an apparatus and a system for disinfecting air and/or surfaces.

### Technological background

Hypochlorous acid (HOCI) is a weak acid that forms when chlorine dissolves in water, and itself partially dissociates, forming hypochlorite ClO⁻. Hypochlorous acid (HOCI) and ClO⁻ are oxidizers, and the primary disinfection agents of chlorine solutions.

HOCI is an endogenous substance in all mammals and is effective against a broad range of microorganisms. Neutrophils, eosinophils, mononuclear phagocytes and B-lymphocytes produce HOCI in response to injury and infection through the mitochondrial membrane-bound enzyme known as "respiratory burst nicotinamide adenine dinucleotide phosphate oxidase". In aqueous solution, HOCI dissociates into H⁺ and ClO⁻, denaturing and aggregating proteins. HOCI also destroys viruses by chlorination by forming chloramines and nitrogen centered radicals, resulting in single- as well as a double-stranded DNA breaks, rendering the nuclear acid useless and the virus harmless.

HOCI has been used, for example, in eye care, where it can provide effective relieve from dry eyes and hordeola. HOCI has also been used to neutralize odors, for water treatment in water supply systems or for the treatment of wounds.

Microorganisms, in particular viruses and bacteria, are present everywhere in everyday life. Some of these viruses and microorganisms can be harmful to mammals in general and to humans in particular. With the COVID-19 epidemic caused by the SARS-CoV-2 virus the need for methods and systems for the disinfection of air and surfaces has grown even more.

JP H11-169441 A discloses an indoor sterilization method capable of simultaneously sterilizing not only ceilings and walls but also the air by atomization of a disinfectant. The disinfectant is sterilized water containing hypochlorite with a pH between 3 and 7.5 and having a hypochlorous acid concentration of 10 to 200 ppm.

JP 2007-162997 A discloses a dry fog generator suitable for a waiting room or a patient's room of a hospital, for sterilizing or sanitizing floating fungus by nebulizing and emitting a chemical. The chemical is a hypochlorite solution, which is sprayed from a nozzle of the dry fog generator.

Prior art document US 2014/0119992 A1 discloses a disinfecting solution comprising a liquid having an HOCI concentration between 50 ppm and 4.000 ppm and a quantity of ethyl alcohol comprising between 0.05% and about 10% of the disinfectant solution by weight.

WO 2011/085470 A1 provides a delivery mechanism for dispersing a pathogen disinfectant liquid into the air or onto surfaces. The apparatus includes a container comprising a sunlight-resistant compartment containing a pathogen disinfectant liquid, a propellant, and an actuator coupled to a valve for opening and closing the valve, wherein opening of the valve causes the propellant to move at least a portion of the pathogen disinfectant liquid through an orifice in the container.

Prior art document CN 111481723 A discloses a method for disinfecting air in gathering places by using a weak hypochlorous acid disinfectant wherein the weak hypochlorous acid disinfectant has a pH of 5.0 to 6.5 and contains 10 ppm to 500 ppm hypochlorous acid and 10 ppm to 9.000 ppm sodium chloride. A cold fogging machine using high frequency ultrasonic vibrations between 5 kHz to 1.7 MHz disperses the weak hypochlorous acid disinfectant into the air at a rate of 0.5 to 1.5 l/hour.

While the dispersion of disinfectants containing HOCI into the air with dispersion rates between 0.5 to 1.5 l/hour is useful for disinfection of air, these high dispersion rates have the disadvantage, that the disinfectant is emitted as a dense fog. The emission of such dense fogs is not only visually unpleasing, but it also prevents a continuous dispersion of the disinfectant over long periods of time, when animals or humans are present. At dispersion rates of 0.5 to 1.5 l/hour the humidity air is increased to almost 100 % and the disinfectant can be damaging to equipment and surfaces present in the volume or area. Therefore, in the prior art the disinfectants are emitted in short bursts, the bursts being distributed over the course of the day.

### Disclosure of the invention: problem, solution, advantages

It is an object of the present invention to provide a method for disinfecting air and/or surfaces which can be carried out truly continuously, in particular for essentially 24 hours a day, with people and/or animals present.

To solve the object the invention proposes a method for disinfecting air and/or surfaces, wherein a disinfectant is dispersed continuously into a volume and/or area to be disinfected, wherein the disinfectant comprises electrolyzed and/or sterilized water containing hypochlorous acid, wherein the disinfectant is dispersed at a dispersion rate between 0.5 ml/m³/h and 12 ml/m³/h.

The unit m³ in the dispersion rate relates to the volume and/or area to be disinfected.

In the inventive method the disinfectant is dispersed at a dispersion rate between 0.5 ml/m³/h and 12 ml/m³/h. The disinfectant is therefore not dispersed as a dense fog or mist. Because of the dispersion rate the disinfectant can be distributed essentially homogeneously in the volume and/or area to be disinfected. With a dispersion rate between 0.5 ml/m³/h and 12 ml/m³/h the dispersed disinfectant is not noticeable by humans or animals present. Advantageously, the disinfectant dispersed into the volume and/or area stays afloat for a long time and continuously settles on any surfaces present in the volume and/or area, thereby disinfecting both the air and the surfaces present in the volume and/or area. When the disinfectant settles on surface in the volume and/or area, additional disinfection protection for up to 96 hours is provided.

Furthermore, because of the low dispersion rate between 0.5 ml/m³/h and 12 ml/m³/h the disinfectant can be dispersed truly continuously into the volume and/or area for at least 6 hours a day, preferably for at least 12 hours a day, further preferably for at least 16 hour a day, still further preferably for essentially 24 hours a day.

The disinfectant comprising electrolyzed and/or sterilized water containing hypochlorous acid is not harmful to humans or animals. Therefore, when dispersed at the low dispersion rate of 0.5 ml/m³/h and 12 ml/m³/h, the air inside the volume and/or area can be inhaled without disturbing humans or animals and without causing any harm. To the contrary, the disinfectant comprising electrolyzed and/or sterilized water containing hypochlorous acid has beneficial health effects.

The method of the present invention is the first truly continuous disinfection method for air and surfaces.

The disinfectant, in particular the electrolyzed and/or sterilized water, can be positively charged electrolyzed water (EW+) or negatively charged electrolyzed water (EW-).

The method can be carried out in areas with or without humans or animals present. The method may be carried out in gathering places, office buildings, schools, universities, private homes and open areas such as parks or sporting stadiums. The method may furthermore be carried out in any public, retail or industrial locations, in railway stations, concert halls, malls, administrative buildings, hotels, hospitals, retirement homes, and in the meat, milk, or egg production.

The method maybe furthermore carried out in the veterinary field or in the field of animal husbandry, livestock farming or animal breeding. Advantageously, when carried out in these fields the amount of antibiotics and antiviral substances can significantly be reduced compared to the prior art.

Preferably, the dispersion rate lies between 0.75 ml/m³/h and 7.5 ml/m³/h, further preferably between 1.0 ml/m³/h and 3.0 ml/m³/h, still further preferably between 1.5 ml/m³/h and 2.0 ml/m³/h.

By selecting a suitable dispersion rate, the method can be carried out in various areas and fields of applications. For example, when the method is carried out in closed gathering places a lower dispersion rate between 0.5 ml/m³/h and 1.5 ml/m³/h may be chosen compared to open gathering spaces. When the method is carried out in the field of animal husbandry a higher dispersion rate may be suitable, for example a dispersion rate between 3.0 ml/m³/h and 7.5 ml/m³/h maybe chosen.

In the scope of the invention, any value of the dispersion rate between 0.5 ml/m³/h to 12 ml/m³/h can be selected. Furthermore, lower limits and/or upper limits of the cited ranges for the dispersion rate may be combined to adapt the method for the specific applications. For example, it is within the scope of the invention that the dispersion rate is between 7.5 ml/m³/h and 12 ml/m³/h or between 0.5 and 1.0 ml/m³/h, etc.

Preferably, the disinfectant is dispersed, in particular without interruption, for at least 12 hours a day, further preferably for at least 16 hours a day, still further preferably for at least 20 hours a day, particularly preferably for essentially 24 hours a day. In particular, the disinfectant is dispersed for 24 hours a day. In the method, the disinfectant may also be dispersed for seven days a week.

By dispersing the disinfectant without interruption for at least 12 hours a day and up to 24 hours a day a truly continuous disinfection method is provided which, because of the special disinfectant and the dispersion rate for the disinfectant, can be carried out with or without humans or animals present.

Preferably the volume and/or area to be disinfected is between 3 m³ and 150.000 m³, further preferably between 100 m³ and 50.000 m³, still further preferably between 1.000 m³ and 10.000 m³, particularly preferably between 2.000 m³ and 5.000 m³.

Thus, the method is suitable for disinfection of a large range of volumes and/or areas.

The disinfectant can be dispersed into very small volumes, for example small rooms, and into very large volumes, for example stadiums, convention centers, large office buildings and the like.

Still further preferably the disinfectant is dispersed as an aerosol, wherein a mean value of a droplet size distribution is between 0.5 µm and 50 µm, preferably between 1 µm and 10 µm, further preferably between 2 µm and 8 µm, particularly preferably between 3 µm and 7 µm.

By dispersing the disinfectant as an aerosol comprising droplets with sizes of about 0.5 to 50 µm, the disinfectant droplets can stay afloat for a long time, e.g. up to 6 hours, in the air inside the volume and/or area. Thus, the disinfectant can diffuse homogeneously throughout the volume and/or area to be disinfected. Because of gravity the droplets of the disinfectant, or of the aerosol of the disinfectant, will continuously and slowly sink down and eventually settle on the surfaces present in the volume or area, providing an additional disinfection for up to 96 hours.

Further preferably, the disinfectant has a pH between 5 and 9, preferably between 6 and 8, further preferably between 6.5 and 7.5.

The disinfectant of the method is preferably neutral or only slightly acidic or basic. This allows for the disinfectant to be inhaled by humans or animals without harm.

Preferably, the disinfectant does not comprise sodium, sodium chloride or dissolved sodium chloride.

Since the disinfectant is not salty, the risk of corrosion of surfaces in the volume and/or area is greatly reduced.

Preferably, the hypochlorous acid has a concentration in the disinfectant between 10 ppm and 1.000 ppm, further preferably between 100 ppm and 500 ppm, still further preferably between 200 ppm and 400 ppm.

Further preferably the disinfectant comprises at least one additive.

By adding at least one additive, further beneficial effects can be obtained. For example, the additives may increase the disinfection capability of the disinfectant used in the method. Also, the additives may be odors, which improve the room quality and are pleasant to persons present. Furthermore, the additives may be stabilizers that stabilize the disinfectant, in particular the hypochlorous acid in the disinfectant.

There may be one or more additives present, which can be chosen independently from each other.

Further preferably the additive is present in the disinfectant with a weight percentage or a volume percentage between 0.1 % and 5.0 %, preferably between 0.25 % and 1.0 %, further preferably between 0.5 % and 0.75 %.

If more than one additive is present, each of the additives may be present in the disinfectant with a weight percentage or a volume percentage between 0.1 % and 5.0 %, preferably between 0.25 % and 1.0 %, further preferably between 0.5 % and 0.75 %.

Still further preferably the at least one additive comprises an essential oil, wherein the essential oil further preferably is at least one out of cinnamon oil, carvacrol oil, thymol oil, cajuput oil, clove oil, eucalyptus oil, sage oil, tea tree oil.

The use of essential oils is particularly beneficial since essential oils may have themselves a disinfecting effect, for example thymol oil. In addition, essential oils provide a nice and pleasant odor. For different open or closed spaces or volumes and/or for different times of the day and/or for different applications, different additives, in particular different essential oils, can be selected.

While it is preferred, that the essential oils are contained in the disinfectant, it is also possible, that the essential oils are dispersed separately, for example with a separate fogging generator, via a heating, ventilation and air conditioning system (HVAC) or by any other suitable means.

Furthermore, the additives may comprise a mixture of different essential oils.

Still further preferably the disinfectant has a redox potential between 500 mV and 1.500 mV, preferably between 700 mV and 1.200 mV, further preferably between 900 mV and 1.000 mV.

Because of the advantageous redox potential the disinfectant can be inhaled without any harmful impact on humans or animals. Furthermore, the disinfectant does not have disadvantageous effects on surfaces such as corrosion or the like.

It is still further preferred that the disinfectant is dispersed via a heating, ventilation and air conditioning system (HVAC). The use of an HVAC system to disperse and distribute the disinfectant allows for the disinfectant to be dispersed in a large building or throughout multiple rooms at the same time.

Preferably, the air of the volume and/or area is filtered. By filtration of the air of the volume and/or area, impurities in the air can be reduced. Particularly preferably, the filtration can be a low MERV7-11 or a H7-11 filtration. Further preferably, an activated carbon filter with or without an iodine filter, or a cold catalyst filter can be used. Still further preferably, a positive ions or plasma generator, MERV 12-16 and/or HEPA filters may be used. It is also possible that the operation of an UV lamp or an ozone generator is combined with the dispersion of the disinfectant.

The filtration of the air of the volume and/or area can be carried out by an HVAC system or with an independent air purification system.

It is possible, that the dispersed disinfectant, in particular the aerosol of the disinfectant, is not filtered, but that the dispersion of the disinfectant and the filtration of the air are carried out independently and in parallel.

However, it may be preferred that the dispersed disinfectant passes through the filters of the air purification system or through the filters of the HVAC system. Studies have shown that viruses and bacteria can accumulate on filter materials. Because of this accumulation, cleaning personal cleaning or changing the filters of HVAC systems or air purification systems have been shown to have a higher infection risk from such filters. This problem is particularly prevalent in connection with HVAC and air purification systems in airplanes. It is therefore particularly advantageous that the dispersed disinfectant passes through the filters, for example of HVAC systems or air purification systems, so that the contamination of the filters with pathogens is reduced.

By combining the continuous dispersion of a disinfectant into a volume and/or area to be disinfected with the filtering and/or purification of the air of the volume and/or area, a multi-layered disinfection and air purification method is provided, which embellishes existing air purification processes and improves the process of reducing pathogens in the air and/or on surfaces in large and small areas.

Still further preferably the aerosol of the disinfectant stays in the air of the volume and/or area for at least 4 hours, preferably for at least 5 hours, further preferably for at least 6 hours.

Preferably a concentration of at least one type of virus and/or bacteria in the volume and/or area, preferably on a surface in the volume and/or area, is reduced by a log reduction value between 2-log and 8-log, preferably between 3-log and 7-log, further preferably between 4-log and 6-log, still further preferably between 4-log and 5-log.

Thus, with the method between 99 % to 99.999999 % of bacterial and/or viruses can be neutralized. Particularly preferably between 99.99 % and 99.999 % of bacteria or viruses can be neutralized.

At least one of the following microorganisms can be neutralized with the method: Aspergillus Niger, Aureobasidium pullulans, Acinetobacter Baumannii, Bacteroides Fragilis, Corynebacterium Diphtheriae, Clostridium Difficile (spores), Candida Albicans, Corynebacterium Amycolatum, Enterbacter Aerogenes, Escherichia Coli, Enterococcus Faecalis - VRE MDR, Hemophilus Influenzae, Klebsiella Pneumoniae, Klebsiella Oxytoca, Malassezia Furfur, Micrococcus Yunnanesis, Methicillin- Resistant Staphylococcus Aureus, Micrococcus Luteus, Proteus Mirabilis, Pseudomonas Aeruginosa, Serratia Marcescens, Staphylococcus Aureus - MSRA, Staphylococcus Epidermidis, Staphylococcus Haemolyticus, Staphylococcus Hominis, Staphylococcus Saprophyticus, Trichophyton Mentagrophytes, Vancomycin Resistant Enterococcus Faecium, Vibrio Vulnificus, Salmonella, MRSA, Avian influenza virus (AIV), H7N1, Sars CoV-2 (Covid 19), Sars CoV-1, Mers, Noroviruses.

It is still further preferred that the dispersion rate is adjusted based on at least one of the parameters: concentration of hypochlorous acid in the disinfectant, air exchange rate, humidity, atmospheric pressure, surface area and/or material of surfaces present in the volume and/or area, decay rate of disinfectant, number of people present in the volume and/or area.

The air exchange rate can be the exchange rate of the air inside the volume and/or area to be disinfected, the humidity can be the room humidity. By, preferably dynamically, adjusting the dispersion rate the concentration levels of the disinfectant in the air and/or on the surfaces in the volume and/or area to be disinfected can controlled within the optimum ranges to neutralize pathogens during the continuous dispersion process.

Preferably the disinfectant for carrying out the method is externally provided as a liquid. However, it is also possible that the HOCI is provided as a concentrate, as granules or in the form of tablets and that the method comprises the step of diluting or dissolving said concentrate, granules or tablets in water, preferably tap water.

Another solution to the object of the invention is the provision of an apparatus for disinfecting air and/or surfaces, in particular for dispersing disinfectants, for use in a method as described above, comprising a housing with an outlet opening and a nozzle, wherein the apparatus is configured to eject and/or disperse droplets, in particular an aerosol, of a disinfectant, wherein the apparatus comprises an air pump, in particular a Venturi pump, employing Bernoulli's principle to pump a disinfectant through the nozzle.

The apparatus of the invention can be used in the method as described above. Any features or functions described above in connection with the inventive method can be applied to the apparatus accordingly, where applicable.

In contrast to the known prior art ultrasonic dispersers for disinfectants in the apparatus of the present invention a disinfectant is syphoned, preferably from a disinfectant reservoir, and pumped through the nozzle by using an air pump employing Bernoulli's principle. The use of an air pump allows for a precise control of pressure, droplet sizes and dispersion rates of the disinfectant.

While prior art dry or cold fogging machines are only capable of producing a dense fog or mist, the apparatus according to the invention produces a fine fog of the disinfectant and disperses the disinfectant at dispersion rates which allow for a continuous disinfection for up to 24 hours a day with or without people present in the volume and/or area to be disinfected.

Preferably, the apparatus is configured to disperse a disinfectant at a dispersion rate between 0.5 ml/m³/h and 12 ml/m³/h. In combination with a disinfectant comprising electrolyzed and/or sterilized water containing hypochlorous acid, an unperceivable ultrafine fog of disinfectant can be dispersed, which is breathable and can be inhaled by humans or animals without any harm to the humans or animals.

By using an air pump, the disinfectant can be pumped through the nozzle at high velocities, which prevents clogging of the nozzle.

The apparatus may comprise more than one air pump and/or more than one nozzle. For example, to disperse the disinfectant into a large volume and/or area, such as a cinema, a high power air pump can be used. However, a high power air pump may cause an increased noise level. Therefore, it can be advantageous that the apparatus comprises more than one of the smaller air pumps. Preferably the apparatus comprises at least two air pumps and/or nozzles, further preferably the apparatus comprises at least four air pumps and/or nozzles, still further preferably the apparatus comprises at least six air pumps and/or nozzles. When more than one air pump is used, the number of air pumps operated simultaneously may vary depending on the disinfection requirements.

Preferably the apparatus further comprises a micro sieve arranged essentially at the outlet opening, wherein the micro sieve has openings, wherein a diameter of the openings is less than 10 µm, preferably less than 5 µm, further preferably less than 3 µm, still further preferably less than 1 µm.

By arranging a micro sieve essentially at the outlet opening, the disinfectant pumped through the nozzle by the air pump can be pushed onto the micro sieve, in particular at a high velocity, so that the disinfectant is dispersed into a very fine aerosol by the micro sieve, where a mean droplet size of the produced droplets is between 0.5 µm to 50 µm, preferably between 1 µm to 10 µm, further preferably between 2 µm to 8 µm and in particular preferably between 3 µm and 7 µm.

Thus, the apparatus is preferably configured such that a disinfectant pumped through and ejected from the nozzle subsequently passes through the micro sieve to form droplets, in particular an aerosol.

Preferably the housing comprises a cavity, wherein the micro sieve is disposed in the cavity and wherein a mouth of the nozzle opens into the cavity.

A disinfectant pumped through the nozzle by the air pump is ejected from the mouth of the nozzle into the cavity. On the opposing side of the nozzle, the cavity opens to the outside through the outlet opening of the housing. The micro sieve is arranged preferably in the cavity, but close to the outlet opening so that a disinfectant ejected from the nozzle is pushed against the micro sieve, dispersed into a fine aerosol and ejected from the apparatus through the outlet opening.

The mouth of the nozzle may also be called an orifice.

Still further preferably, the mouth of the nozzle is configured to eject the disinfectant into a spray cone with an opening angle of at least 140°, preferably at least 150°, further preferably at least 160°.

For example, the mouth of the nozzle may have a conical shape with an opening angle of at least 140°, preferably at least 150°, further preferably at least 160°.

Still further preferably, the housing comprises a return passage, which fluidically connects to the cavity, wherein the return passage is configured to return disinfectant pooling in the cavity and/or not being ejected from the apparatus back to a disinfectant reservoir.

Disinfectant not being ejected from the apparatus may pool in the cavity between the nozzle and the micro sieve or the outlet opening. By providing a return passage, any unused disinfectant can be returned to a disinfectant reservoir. This prevents clogging of the apparatus.

The disinfectant reservoir may be part of the apparatus.

Still further preferably the nozzle comprises a rotation nozzle piece, wherein the rotation nozzle piece is configured to be rotated by operation of the air pump.

In particular, the rotation nozzle piece may comprise the mouth of the nozzle. By operation of the air pump the rotating nozzle piece pump is rotated. Disinfectant pumped though the nozzle is dispersed out of the mouth of the nozzle and accelerated in the radial direction by centrifugal forces from the rotation nozzle piece.

The mouth, also called orifice, of the nozzle, which is preferably disposed in the rotation nozzle piece, preferably has an opening diameter of less than 3.0 mm, further preferably of less than 1.0 mm, particularly preferably of less than 0.5 mm, still further preferably of less than 0.3 mm, further preferably of less than 0.25 mm, particularly preferably of less than 0.1 mm.

Furthermore preferably, the housing comprises a connecting section for connecting the apparatus to a disinfectant reservoir.

The connecting section can be connected to a valve of the disinfectant reservoir and by operation of the air pump a disinfectant is pumped out of the disinfectant reservoir through the nozzle and pushed against the micro sieve so that an aerosol of fine droplets of the disinfectant is ejected from the outlet opening of the apparatus.

Still further preferably the apparatus further comprises at least one sensor, wherein preferably the at least one sensor comprises a pump pressure sensor, a humidity sensor, a temperature sensor, an atmospheric pressure sensor, a dispersion rate sensor, a flow rate sensor, a reservoir level sensor.

With data from the at least one sensor, the dispersion rate can be adjusted to the specific application of use of the apparatus.

Preferably, the disinfectant reservoir of the apparatus comprises a connector for a water tap and a micro dosing system, in particular a micro dosing multi-channel system. Tap water can be filled into the disinfectant reservoir through the connector and the disinfectant can be produced by diluting or dissolving HOCI in the form of concentrates, granules or in the form of tablets in the tap water inside the disinfectant reservoir. The HOCI concentrate, granules or tablets are delivered into the water by the micro dosing system.

Furthermore, the concentration of HOCI in the disinfectant stored in the disinfectant reservoir can be monitored using appropriate sensors. The sensors may be sensors to measure the concentration of HOCI in the disinfectant and/or in the air. The micro dosing system can comprise, preferably multiple, peristaltic pumps in order to transfer the exact quantity of the concentrate, granules or tablets to the disinfectant reservoir.

The disinfectant reservoir may also comprise an automatic stirring devices to ensure even concentration of HOCI in the disinfectant at all times.

This system, i.e. the disinfectant reservoir or the apparatus comprising the disinfectant reservoir, can be a closed loop system made from hospital grade Stainless Steel and coated with anti-microbial ZnO film to protect against any build-up of germs or pathogens on the exposed surfaces.

Another solution to the object of the invention is the provision of a disinfectant for a method for disinfecting air and/or surfaces as described above, wherein the disinfectant comprises electrolyzed and/or sterilized water containing hypochlorous acid, wherein the disinfectant further comprises at least one additive, wherein the at least one additive preferably comprises an essential oil, in particular cinnamon oil, carvacrol oil, thymol oil, cajaput oil, clove oil, eucalyptus oil, sage oil or tea tree oil.

A further solution to the problem solved by the invention is the provision of a system for disinfecting air and/or surfaces, configured for carrying out a method as described above, wherein the system comprises at least one sensor and a data processing unit, wherein the data processing unit is configured to receive measurement data from the sensor, wherein the data processing unit is configured to, preferably dynamically, adjust a dispersion rate of a disinfectant based on the measurement data.

The data processing unit, for example a microprocessor or a computer or the like, receives measurement data from the sensors. The sensors can be part of an apparatus as previously described or they can be arranged in a volume and/or area to be disinfected.

The data processing unit receives data from the sensors and adjust the dispersion rate of the disinfectant into the volume and/or area to be disinfected. Particularly preferably the data processing unit adjusts the dispersion rate to between 0.5 to 12 ml/m³/h, further preferably between 0.75 ml/m³/h and 7.5 ml/m³/h, still preferably 1.0 ml/m³/h and 3.0 ml/m³/h, particularly preferably between 1.5 ml/m³/h and 2.0 ml/m³/h.

Preferably, the system comprises a heating, ventilation and air conditioning system (HVAC) configured to distribute and/or disperse the disinfectant.

It is furthermore preferred that the system comprises an apparatus as described above.

A still further solution to the problem is the provision of a disinfectant reservoir, comprising a container, wherein the container has an interior coating, preferably of ZnO, and/or wherein the container has an exterior coating, preferably of ZnO, further preferably a lamination of a ZnO film.

Still further preferably the container of the disinfectant reservoir is a double-walled container.

The container can be made from hospital grade stainless steel.

The disinfectant reservoir may be part of or combined with an apparatus as described above.

Yet another solution to the problem is the provision of a computer program product comprising instructions which, when the program is executed by a computer, cause the computer to carry out an adjustment of a dispersion rate in a method as described above based on the measurement data of at least one sensor.

The computer program product may comprise a mobile application, which can be carried out on a portable device such as a smartphone, a smartwatch, a tablet and so forth.

Preferably, a user may use the mobile application in order to adjust the parameters of a system for disinfecting air and/or surfaces as described above. For example, the user may adjust the dispersion rate, or he may adjust parameters such as the room humidity or he may specify surfaces present in the volume and/or area.

### Brief description of the figures

The invention is described in the following with reference to the accompanying figures.
Fig. 1 shows a system for disinfecting air and/or surfaces,
Fig. 2a shows a cut out view of an apparatus for dispersing a disinfectant,
Fig. 2b shows an exploded view of an apparatus for dispersing a disinfectant,
Fig. 2c shows a cross sectional view of an apparatus for dispersing a disinfectant,
Fig. 3 shows a nozzle for an apparatus for dispersing a disinfectant, and
Fig. 4 shows an apparatus for dispersing a disinfectant mounted on a disinfectant reservoir.

### Detailed description of the figures

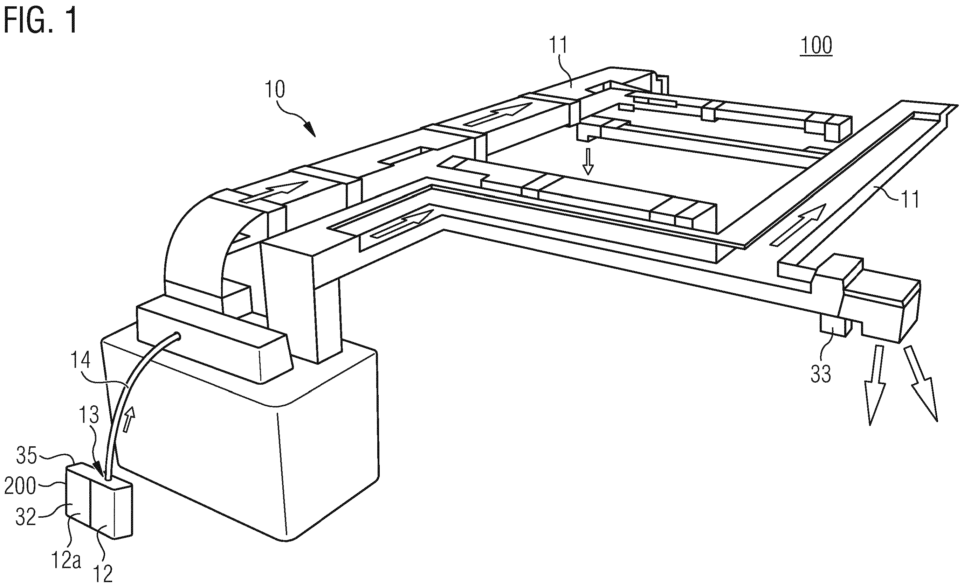

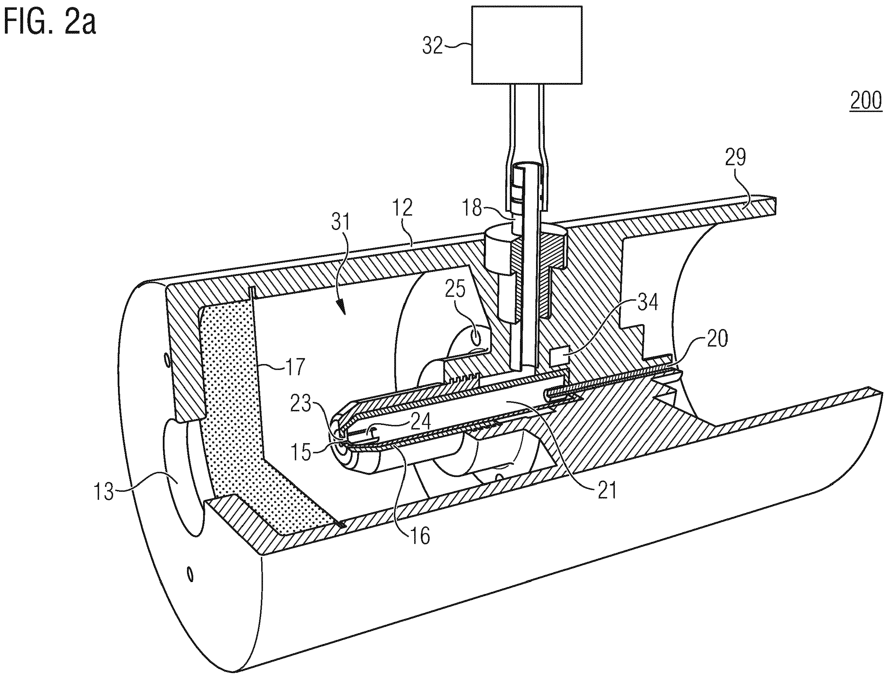

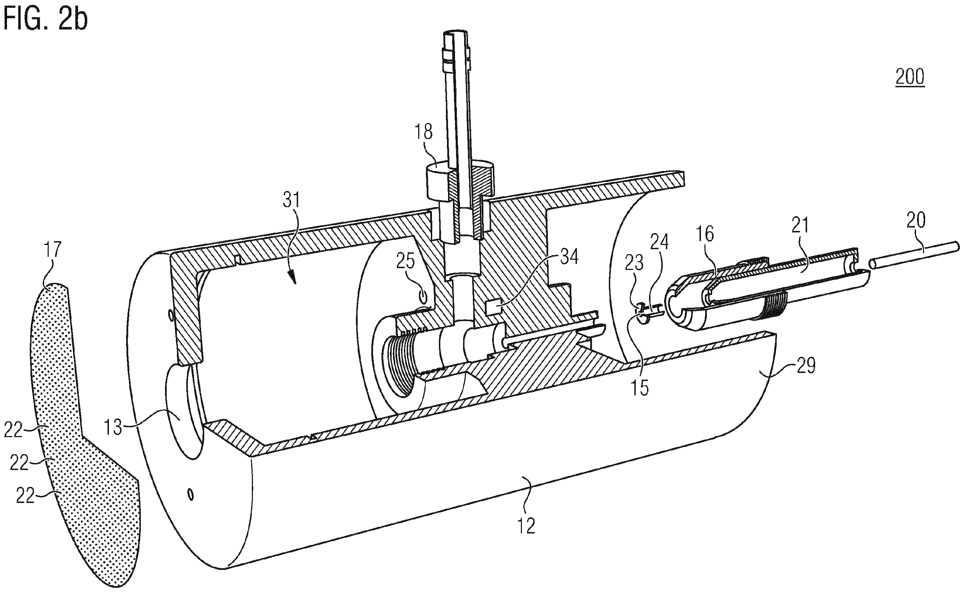

Fig. 1 shows a system 100 for disinfecting air and/or surfaces. The system 100 comprises an apparatus 200 for dispersing a disinfectant as well as an HVAC system 10 comprising ducts 11. The apparatus 200 has a shell 12a. Inside the shell 12a housing 12 comprising an outlet opening 13, as shown in Figs. 2a and 2b, is disposed. Furthermore, an air pump 32 is arranged inside shell 12a. An aerosol of a disinfectant is ejected through the outlet opening 13 of the apparatus 200 and guided to the HVAC system 100 via a delivery tube 14. During operation of the system 100, the aerosol of the disinfectant is distributed via the ducts 11 throughout a building. The system further comprises a sensor 33 for measuring, for example atmospheric pressure or room humidity. A data processing unit 35 is disposed inside shell 12a. Based on measurement data of the sensor 33, and of additional sensors 34 of the apparatus (Figs. 2a and 2b), the data processing unit adjusts a dispersion rate of the disinfectant.

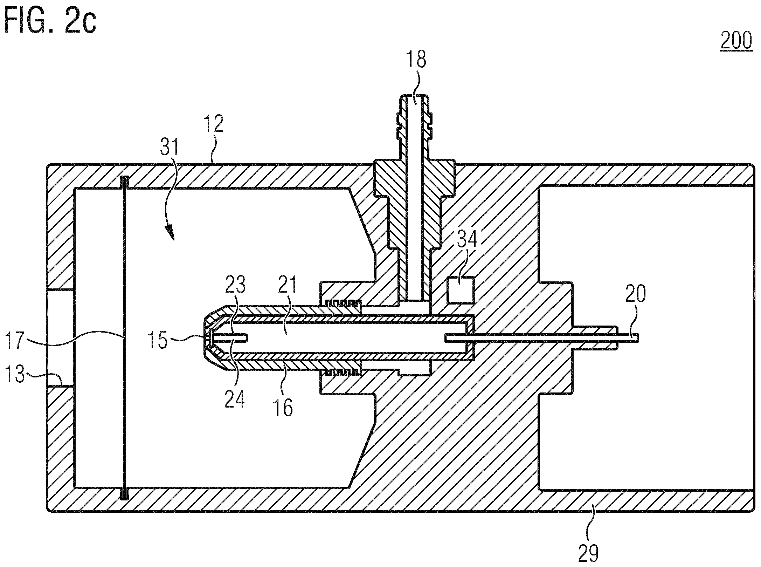

Fig. 2a shows a cut out view of an apparatus 200 for dispersing a disinfectant, which can be used in the system 100 of Fig. 1. Fig. 2b shows an exploded view of the apparatus of Fig. 2a. Fig. 2c shows a cross sectional view of the apparatus of Figs. 2a and 2b.

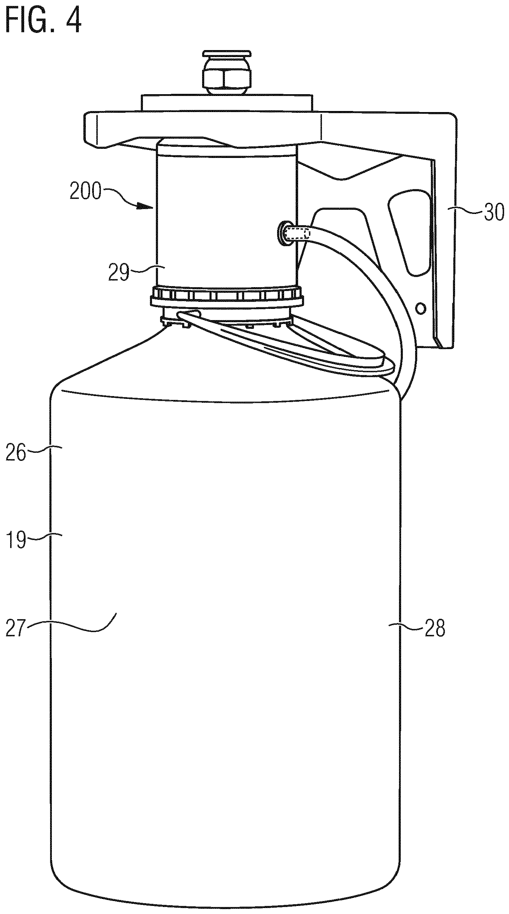

The apparatus 200 comprises a housing 12 having an outlet opening 13. A cavity 14 is disposed inside the housing 12. A mouth 15 of a nozzle 16 opens into the cavity 31. A micro sieve 17 is arranged essentially at the outlet opening 13. The housing 12 further comprises a connector 18 for an air pump 32. When the air pump 32 is operated, a low pressure region is created inside the nozzle 16 and a disinfectant is syphoned from a disinfectant reservoir 19 (Fig. 4) through an inlet pipe 20. The disinfectant is mixed with air in a mixing chamber 21 of the nozzle 16 and ejected from the mouth 15 of the nozzle 16 into the cavity 31. Because of the high ejection velocity of the disinfectant from the nozzle 16, the disinfectant is pushed against the micro sieve 17. The micro sieve 17 has openings 22 with a diameter of about 1 µm to 10 µm. Disinfectant passing the micro sieve 17 is dispersed into an aerosol with droplet sizes in the range of preferably 3 µm to 7 µm. The aerosol is then ejected through the outlet opening 13 either directly into a volume and/or area to be disinfected or the aerosol is guided via a delivery tube 14 into the ducts 11 of an HVAC system 100 (Fig. 1). The nozzle 16 further comprises a rotation nozzle piece 23 configured as an atomization device 24. The rotation nozzle piece 23 comprises the mouth 15 of the nozzle 16. The rotation nozzle piece 23 is mounted rotatably in the nozzle 16 and is rotated by the operation of the air pump 32. Because of the rotation of the rotation nozzle piece 23 the disinfectant ejected from mouth 15 of nozzle 16 is accelerated in the radial direction to form a spray cone with an opening angle of approximately 160°. The apparatus 200 also comprises a sensor 34, configured for measuring a flow rate of the disinfectant through the nozzle or to measure the air pump pressure.

The housing 12 further comprises a return passage 25 fluidically connected to the cavity 31 and the disinfectant reservoir 19 (Fig. 4). Any disinfectant pooling inside cavity 31 is returned to the disinfectant reservoir 19 via return passage 25.

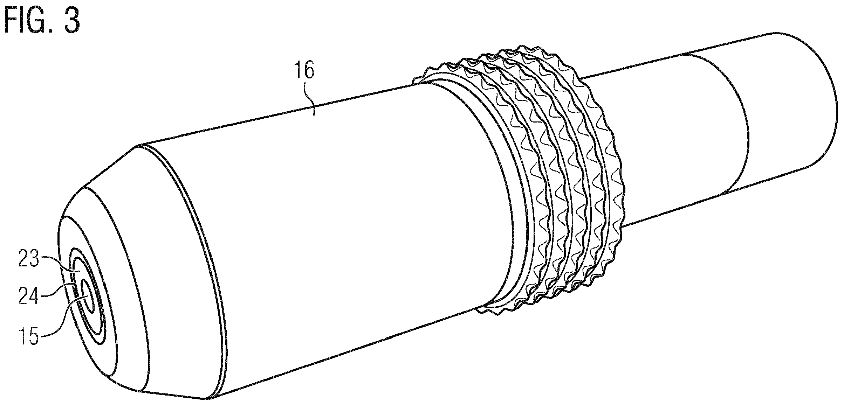

Fig. 3 shows a perspective view of the nozzle 16 of the apparatus 200. The nozzle 16 comprises a rotation nozzle piece 23 configured as an atomization device 24. The mouth 15 of the nozzle 15 is disposed in the rotation nozzle piece 23. A disinfectant syphoned from a disinfectant reservoir 19 and pumped through nozzle 16 is ejected from mouth 15 of the rotation nozzle piece 23 into a spray cone with an opening angle of about 160°.

Fig. 4 shows the apparatus 200 mounted on top of a disinfectant reservoir 19. The disinfecting reservoir 19 is configured as a container 26 having an exterior coating 27 of a ZnO laminate. On the inside the container 26 is also covered with ZnO. The container 26 is configured as a double-walled container 28. The apparatus 200 is connected to the disinfectant reservoir 19 via a connecting section 29 (Figs. 2a and 2b). The disinfectant reservoir 19 also comprises a latch 30 for mounting the disinfectant reservoir to a wall.

### List of reference numerals

- 100: System for disinfecting air and/or surfaces
- 200: Apparatus for dispersing a disinfectant
- 10: HVAC system
- 11: Duct
- 12: Housing
- 12a: Shell
- 13: Outlet opening
- 14: Delivery tube
- 15: Mouth
- 16: Nozzle
- 17: Micro sieve
- 18: Connector
- 19: Disinfectant reservoir
- 20: Inlet pipe
- 21: Mixing chamber
- 22: Opening
- 23: Rotation nozzle piece
- 24: Atomization device
- 25: Return passage
- 26: Container
- 27: Exterior coating
- 28: Double-walled container
- 29: Connecting section
- 30: Latch
- 31: Cavity
- 32: Air pump
- 33: Sensor
- 34: Sensor
- 35: Data processing unit

## Claims

1. Method for disinfecting air and/or surfaces, wherein a disinfectant is dispersed continuously into a volume and/or area to be disinfected, wherein the disinfectant comprises electrolyzed and/or sterilized water containing hypochlorous acid, **characterized in that** the disinfectant is dispersed at a dispersion rate between 0.5 ml/m³/h and 12 ml/m³/h.

2. Method according to claim 1, **characterized in that** the dispersion rate is between 0.75 ml/m³/h and 7.5 ml/m³/h, preferably 1.0 ml/m³/h and 3.0 ml/m³/h, further preferably between 1.5 ml/m³/h and 2.0 ml/m³/h, and/or that the disinfectant is dispersed for at least 12 hours a day, preferably for at least 16 hours a day, further preferably for at least 20 hours a day, particularly preferably for essentially 24 hours a day.

3. Method according to claim 1 or 2, **characterized in that** the disinfectant is dispersed as an aerosol, wherein a mean value of a droplet size distribution is between 0.5 µm and 50 µm, preferably between 1 µm and 10 µm, further preferably between 2 µm and 8 µm, particularly preferably between 3 µm and 7 µm, and/or that the disinfectant has a pH between 5 and 9, preferably between 6 and 8, further preferably between 6.5 and 7.5, and/or that the hypochlorous acid has a concentration in the disinfectant between 10 ppm and 1.000 ppm, preferably between 100 ppm and 500 ppm, further preferably between 200 ppm and 400 ppm, and/or that the disinfectant has a redox potential between 500 mV and 1.500 mV, preferably between 700 mV and 1.200 mV, further preferably between 900 mV and 1.000 mV.

4. Method according to any one of the preceding claims, **characterized in that** the disinfectant comprises at least one additive, wherein the additive preferably is present in the disinfectant with a weight percentage or a volume percentage between 0.1 % and 5.0 %, further preferably between 0.25 % and 1.0 %, still further preferably between 0.5 % and 0.75 %, and/or wherein preferably the at least one additive comprises an essential oil, wherein the essential oil further preferably is at least one out of cinnamon oil, carvacrol oil, thymol oil, cajuput oil, clove oil, eucalyptus oil, sage oil, tea tree oil.

5. Method according to any one of claims 3 to 4, **characterized in that** the aerosol of the disinfectant stays in the air of the volume and/or area for at least 4 hours, preferably for at least 5 hours, further preferably for at least 6 hours.

6. Method according to any one of the preceding claims, **characterized in that** the dispersion rate is adjusted based on at least one of the parameters: concentration of hypochlorous acid in disinfectant, air exchange rate, humidity, atmospheric pressure, surface area and/or material of surfaces present in the volume and/or area, decay rate of disinfectant, number of people present in volume and/or area.

7. Apparatus (200) for disinfecting air and/or surfaces, in particular for dispersing disinfectants, for use in a method according to any one of claims 1 to 6, comprising a housing (12) with an outlet opening (13) and a nozzle (16), wherein the apparatus is configured to eject and/or disperse droplets, in particular an aerosol, of a disinfectant, **characterized in that** the apparatus comprises an air pump (32), in particular a Venturi pump, employing Bernoulli's principle to pump a disinfectant through the nozzle (16).

8. Apparatus (200) according to claim 7, **characterized in that** the apparatus further comprises a micro sieve (17) arranged essentially at the outlet opening (13), wherein the micro sieve (17) has openings (22), wherein a diameter of the openings (22) is less than 10 µm, preferably less than 5 µm, further preferably less than 3 µm, still further preferably less than 1 µm.

9. Apparatus (200) according to claim 8, **characterized in that** the apparatus is configured such that a disinfectant pumped through and ejected from the nozzle (16) subsequently passes through the micro sieve (17) to form droplets, and/or that the housing (12) comprises a cavity (31), wherein the micro sieve (17) is disposed in the cavity (31) and wherein a mouth (15) of the nozzle (16) opens into the cavity (31).

10. Apparatus (200) according to claim 9, **characterized in that** the housing (12) comprises a return passage (25), which fluidically connects to the cavity (31), wherein the return passage (25) is configured to return disinfectant pooling in the cavity (31) and/or not being ejected from the apparatus back to a disinfectant reservoir (19).

11. Apparatus (200) according to any one of claims 7 to 10, **characterized in that** the nozzle (16) comprises a rotation nozzle piece (23), wherein the rotation nozzle piece (23) is configured to be rotated by operation of the air pump (32), and/or that the apparatus further comprises at least one sensor (34), wherein preferably the at least one sensor (34) comprises a pump pressure sensor, a humidity sensor, a temperature sensor, an atmospheric pressure sensor, a dispersion rate sensor, a flow rate sensor, a reservoir level sensor.

12. Disinfectant for a method according to any one of claims 1 to 6, comprising is electrolyzed and/or sterilized water containing hypochlorous acid, **characterized in that** the disinfectant further comprises at least one additive, wherein the at least one additive preferably comprise an essential oil, in particular cinnamon oil, carvacrol oil, thymol oil, cajuput oil, clove oil, eucalyptus oil, sage oil, tea tree oil.

13. System (100) for disinfecting air and/or surfaces, configured for conducting a method according to any one of claims 1 to 6, **characterized in that** the system comprises at least one sensor (33, 34) and a data processing unit (35), wherein the data processing unit (35) is configured to receive measurement data from the at least one sensor (33, 34), wherein the data processing unit (35) is configured to, preferably dynamically, adapt a dispersion rate of a disinfectant based on the measurement data.

14. Disinfectant reservoir (19), comprising a container (26), wherein the container (26) has an interior coating, preferably of ZnO, and/or wherein the container (26) has an exterior coating (27), preferably of ZnO, further preferably a lamination of a ZnO film, and/or wherein the container (26) is a double-walled container (28).

15. A computer program product comprising instructions which, when the program is executed by a computer, cause the computer to carry out the method of claim 6.
